# FASCICULE DE BREVET EUROPEEN

(11) **EP 2 615 125 B1**
(45) Date de publication et mention de la délivrance du brevet: **07.03.2018**
(21) Numéro de dépôt: 13160893.7
(22) Date de dépôt: 10.07.2009
(51) Int. Cl.: C08J 3/075, G01N 21/33, G01N 21/64, G01N 21/78, G01N 21/79, G01N 33/00, C03B 19/12, C03C 1/00, B01J 20/283, B01J 20/10, C08L 83/08, B01J 20/26, B01J 20/28

(54) **Détecteurs nanoporeux de composés aromatiques monocycliques et autres polluants**
Nanoporöse Detektoren für aromatische monozyklische Verbindungen und anderen Schadstoffen
Nanoporous detectors of monocyclic aromatic compounds and other pollutants

(30) Priorité: 11.07.2008 FR 0854755
(43) Date de publication de la demande: 17.07.2013
(62) Demande divisionnaire de: 09784504.4
(73) Titulaire: COMMISSARIAT A L'ENERGIE ATOMIQUE, 75015 Paris (FR); Centre National de la Recherche Scientifique (C.N.R.S.), 75794 Paris Cedex 16 (FR)
(72) Inventeur: Crunaire, Sabine, 59500 DOUAI (FR); Tran-Thi, Thu-Hoa, 77310 ST FARGEAU-PONTHIERRY (FR)
(74) Mandataire: Cabinet Plasseraud

(56) Documents cités:
- EP-A- 1 566 213
- WO-A1-2006/094392
- FR-A1- 2 890 745
- US-A- 4 017 528
- US-A1- 2003 180 964
- US-A1- 2003 231 984
- US-B1- 6 667 016
- KUNZELMANN U ET AL: "Biosensor properties of glucose oxidase immobilized within SiO2 gels", SENSORS AND ACTUATORS B, ELSEVIER SEQUOIA S.A., LAUSANNE, CH, vol. 39, no. 1-3, 1 mars 1997 (1997-03-01) , pages 222-228, XP004087746, ISSN: 0925-4005
- CALVO-MUNOZ M-L ET AL: "Chemical sensors of monocyclic aromatic hydrocarbons based on sol-gel materials: kinetics of trapping of the pollutants and sensitivity of the sensor", SENSORS AND ACTUATORS B, ELSEVIER SEQUOIA S.A., LAUSANNE, CH, vol. 87, no. 1, 15 novembre 2002 (2002-11-15), pages 173-183, XP004391091, ISSN: 0925-4005

## Description

La présente invention concerne des détecteurs nanoporeux d'aldéhydes et leurs applications.

Les composés aromatiques monocycliques sont des polluants atmosphériques toxiques pour la plupart et même cancérigène pour le benzène. On les trouve dans des milieux comme l'industrie pétrochimique, dans l'industrie chimique (utilisation de solvants), dans les environnements proches d'installations industrielles, dans les milieux professionnels (laboratoires de recherche, laboratoires d'analyse, etc.), dans les carburants de voitures et dans l'air ambiant (évaporation de carburants, combustion incomplète d'énergie fossile, tabagisme, utilisation de produits d'entretien ou pour le bricolage).

Pour des problèmes de suivi de pollution atmosphérique dans les atmosphères polluées ou de surveillance des travailleurs directement exposés à ces composés, il est nécessaire de détecter et de quantifier les aromatiques les plus volatils, appelés communément BTEXMs (Benzène, Toluène, Ethylbenzène, Xylènes (ortho, méta et para), triméthyl (1,2,3 ; 1,2,4 et 1,3,5)-benzènes).

Les Mesures des Hydrocarbures Aromatiques Monocycliques (HAM) se font habituellement dans l'air ambiant et en air intérieur :
Ces mesures sont effectuées en 2 étapes distinctes : prélèvement puis analyse. Sur les lieux de travail, on effectue deux types de mesure : soit l'exposition individuelle du personnel soit la mesure d'ambiance autour d'un point fixe de travail. Le prélèvement peut être soit actif (pompage de l'air au travers d'une cartouche de silice recouverte d'une phase stationnaire) ou passif (par diffusion au travers de la même cartouche sans pompage). L'analyse est dans ces deux cas réalisée en différé au laboratoire.

Pour des prélèvements suivis d'une analyse in situ, il existe des analyseurs en continu. On peut citer les micro-chromatographes portables fonctionnant de façon autonome (prélèvement, concentration, analyse). Ils sont cependant encombrants et nécessitent l'utilisation de divers gaz, azote, air et hydrogène avec un détecteur FID (détecteur à ionisation de flamme) ou d'azote avec un détecteur PID (détecteur par photoionisation), ce dernier étant limité pour des concentrations inférieures à 300 ppbv (*volume part per billion* ou parties par milliards en volume).

Pour répondre à la demande en détecteurs à mesure directe, de nombreux chercheurs ont essayé d'élaborer des capteurs chimiques de BTEXMs ayant une bonne sélectivité.

La difficulté de trouver des molécules-sonde spécifiques pour ces composés vient du fait que ceux-ci sont pratiquement apolaires (moments dipolaires variant entre 0 et 0,3 Debye). Les BTEXMs ne peuvent donc que faiblement interagir avec d'autres molécules par des forces de dispersion ou des forces électrostatiques à courte portée. Les principes de détection rapportés dans la littérature sont essentiellement basés sur ces interactions faibles non sélectives et sur des essais de sélectivité par la taille. On utilise à cette fin les macrocycles aromatiques, les fluorophores sensibles à l'environnement microscopique tel que le Rouge de Nil et des capteurs à base de semi-conducteurs et d'oxydes mixtes. Pour une sélectivité par la taille, des molécules cages dont la cavité est taillée pour accueillir le polluant-cible (exemple : paracycolphanes, calixarènes ou cyclodextrines), ont été proposées, mais ces systèmes ne sont pas sélectifs.

On connait aussi des détecteurs de benzène à base de matrices poreuses de polymères hybrides organique-inorganique à base d'alcoxydes de silicium (Si(OR)ₙR₄₋ₙ avec R=CH₃). Avec des matrices d'épaisseur pouvant varier entre 500 µm et 2 mm, et possédant des nanopores de rayon compris entre 3,5 et 9 Å. Calvo-Muñoz et al. ("Chemical sensors of monocyclic aromatic hydrocarbons based on sol-gel materials: kinetics of trapping of the pollutants and sensitivity of the sensor", Sensors and Actuators B, vol. 87, pp. 173-183), 2002) ont montré qu'il est possible de piéger de façon quasi-irréversible le benzène et le toluène et de les discriminer par leur spectre d'absorption. Avec des blocs monolithiques de 2 mm d'épaisseur, la sensibilité obtenue en laboratoire pour le benzène et le toluène est de 10 ppbv et ceci pour une exposition de 2 heures à un débit de 20 mL.min⁻¹. Pour des débits d'exposition plus élevés de 250 mL.min⁻¹, 60 ppbv peuvent être mesuré en un temps d'exposition de 14 minutes. Le rendement de piégeage du benzène et du toluène dans ces matrices est de 100% pour de faibles concentrations (<10 ppbv) et de faible débit (20 mL.min⁻¹) mais diminue fortement (5 à 6 %) lorsque la concentration est > 1 ppmv (*volume par million* ou partie par million en volume) et le débit est > 50 mL.min⁻¹. Dans tous les cas, le piégeage est quasi irréversible. L'ortho-xylène et le méta-xylène ainsi que les triméthylbenzènes, ne peuvent pas diffuser dans ces matériaux dont les pores sont trop petits (diamètre < 20 Å), ce qui en fait des détecteurs sélectifs pour le benzène, le toluène et le para-xylène uniquement. Même si le piégeage et la mesure sont réalisés en une seule étape, le piégeage des polluants est cependant irréversible.

Depuis 2001, la firme NTT (Nippon Telegraph & Telephone Corp.) travaille sur divers adsorbants poreux sous forme de cubes de silice de diamètre d'environ 2 µm avec des tailles de pores contrôlées, qui servent à piéger les BTEMXs. Voir par exemple Ueno Y., A. Tate, and O. Niwa "Benzene sensor and method for manufacturing same", Brevet EP1712889A1, 18 octobre 2006. Les cubes remplissent une chambre à écoulement microfluidique munie d'un système de chauffage (résistance électrique gravé sur la face arrière) qui servira à la désorption thermique des polluants. Pendant l'étape de pré-concentration, l'air contenant les polluants est pompé au travers de la chambre à écoulement pendant une durée de quelques dizaines de minutes à 1h30 selon la teneur des polluants. Ces derniers sont partiellement piégés dans le matériau poreux. La chambre est ensuite chauffée à 200°C pendant 10 secondes pour désorber les polluants. Les gaz sont transférés par une 2ème pompe dans une chambre optique (cellule en quartz de 2cm ou fibre optique de 12 cm) et sont détectés en utilisant leur absorbance à l'aide d'un spectrophotomètre UV (Soma Optics, Fastevert S-2400). La sélectivité est obtenue par déconvolution spectrale à partir d'une banque de données spectrales des divers BTEMXs. L'ensemble du dispositif de piégeage et de détection est inclus dans une mallette ventilée de dimensions 37,5x20x16 cm pesant 6,7 kg, ordinateur non inclus. La sensibilité du détecteur est de 1 ppbv pour 90 min d'exposition, 50 ppbv pour 50 min d'exposition. Le détecteur est peu sensible à l'humidité relative de l'air entre 40 et 80% d'humidité relative.

L'appareil de la firme NTT possède les critères requis en termes de sensibilité et de rapidité mais le dispositif implique toujours deux étapes dont la pré-concentration et l'analyse. Dans ce cas, un système de chauffage avec une rampe rapide en température est nécessaire pour expulser les polluants piégés dans ces matrices et les transférer dans la chambre d'analyse.

De l'examen des analyseurs commercialisés ou proposés dans la littérature, il apparaît qu'il existe une demande de dispositifs permettant le piégeage et la mesure des Hydrocarbures Aromatiques Monocycliques (HAM) en une seule étape tout en conservant le caractère réversible du processus. De préférence on utiliserait une seule chambre pour l'exposition aux HAMs et leur mesure. Il serait aussi souhaitable de pouvoir procéder à la désorption des gaz sans avoir recours à un dispositif de chauffage.

Les étapes de réalisation des capteurs devraient être les plus courtes possibles, par exemple moins de 24 heures.

La taille et le coût de fabrication des capteurs devraient être des plus réduits.

Le matériau des capteurs devrait permettre une diffusion rapide des polluants et montrer un bon rendement de piégeage des HAMs.

Le piégeage devrait être réversible.

Le système de détection utilisant de tels capteurs devrait pouvoir être miniaturisé tout en gardant un bon rapport signal/bruit.

Pour des mesures sur un lieu de travail, il serait également souhaitable qu'un système de détection possède une bonne autonomie, au minimum de 8h par exemple, ce qui correspond à 1 jour de travail et qu'il soit robuste, d'un faible encombrement, facilement portable par un individu et qu'il soit donc léger.

Or après de longues recherches la demanderesse a découvert un détecteur polyvalent de BTEMXs et de leurs solvants usuels, basé sur l'utilisation de nouveaux matériaux poreux, donnant satisfaction.

La présente demande a pour objet un procédé de piégeage d'aldéhydes comme défini dans la revendication 1. Le matériau sol-gel poreux est essentiellement constitué
- d'unités d'un ou plusieurs premier(s) polyalcoxysilane(s) choisi(s) parmi les composés suivants : le (chlorométhyl)triéthoxysilane ; le 1,3-diméthyltetraméthoxydisiloxane ; l'éthyltriméthoxysilane ; le triéthoxy(éthyl)silane ; le triéthoxyméthylsilane ; le triéthoxy(vinyl)silane ; le triméthoxyméthylsilane ; le triméthoxy(vinyl)silane ; le tétraéthoxysilane ou le tétraméthoxysilane et avantageusement un seul polyalcoxysilane et plus particulièrement le tétraméthoxysilane (TMOS) et
- d'unités d'un ou plusieurs second(s) polyalcoxysilane(s) choisi(s) parmi les composés suivants: le (N-(3-(triméthoxysilyl)propyl)éthylènediamine ; le 3-aminopropyltriéthoxysilane (APTES) et le 3-aminopropyltriméthoxysilane et avantageusement le 3-aminopropyltriéthoxysilane, dans un rapport molaire premier(s) polyalcoxysilane(s) / second(s) polyalcoxysilane de 1/0,01 à 1/1, de préférence de 1/0,01 à 1/0,50, notamment de 1/0,01 à 1/0,30, particulièrement de 1/0,01 à 1/0,15, tout particulièrement de 1/0,02 à 1/0,06 ; de préférence le premier polyalcoxysilane est le TMOS. Il est à noter que le (N-(3-(triméthoxysilyl)propyl)éthylènediamine, le 3-aminopropyltriéthoxysilane (APTES) et le 3-aminopropyltriméthoxysilane comportent au moins une fonction amine primaire.

Le(s) premier (s) polyalcoxysilane(s) est (sont) notamment choisi(s) parmi les composés suivants: le méthyltriméthoxysilane, le tétraéethoxysilane, le méthyltriéthoxysilane et le tétraméthoxysilane (TMOS).

De préférence le matériau sol-gel poreux est essentiellement constitué d'unités d'un ou plusieurs premier(s) polyalcoxysilane(s) et d'unités d'un seul second polyalcoxysilane, et particulièrement d'unités d'un seul premier polyalcoxysilane et d'unités d'un seul second polyalcoxysilane.

Un matériau sol-gel de l'invention peut être préparé essentiellement à partir d'un ou plusieurs premier(s) polyalcoxysilane(s) choisi(s) parmi les composés suivants : (chlorométhyl)triéthoxysilane ; 1,3-diméthyltetraméthoxydisiloxane ; éthyltriméthoxysilane ; triéthoxy(éthyl)silane ; triéthoxyméthylsilane ; triéthoxy(vinyl)silane ; triméthoxyméthylsilane ; triméthoxy(vinyl)silane ; tétraéthoxysilane ou tétraméthoxysilane et avantageusement un seul polyalcoxysilane et plus particulièrement le tétraméthoxysilane (TMOS) et d'unités d'un ou plusieurs second(s) polyalcoxysilane(s) choisi(s) parmi les composés suivants : (N-(3-(triméthoxysilyl)propyl)éthylènediamine ; 3-aminopropyltriéthoxysilane (APTES) et 3-aminopropyltriméthoxysilane et avantageusement le 3-aminopropyltriéthoxysilane, dans un rapport molaire premier(s) polyalcoxysilane(s) / second(s) polyalcoxysilane(s) de 1/0,01 à 1/1, de préférence de 1/0,01 à 1/0,50, notamment de 1/0,01 à 1/0,30, particulièrement de 1/0,01 à 1/0,15, tout particulièrement de 1/0,02 à 1/0,06 ; de préférence le premier polyalcoxysilane est le TMOS.

On préfère des matériaux sol-gel poreux pouvant être préparé essentiellement à partir d'un ou plusieurs premier(s) polyalcoxysilane(s) et d'un seul second polyalcoxysilane, et particulièrement d'un seul premier polyalcoxysilane et d'un seul second polyalcoxysilane.

Les modes de préparation sont exposés ci-après.

Le matériau sol-gel de l'invention est poreux et il a une distribution de taille de pores allant de 10 à 60 angströms, notamment de 20 à 60 angströms et une surface spécifique de 200 à 800 m².g⁻¹. Préférentiellement, la surface spécifique est de 650 ±70 m².g⁻¹.

Parmi les seconds polyalcoxysilanes entrant dans la composition du matériau, on préfère le 3-aminopropyltriéthoxysilane (APTES).

Des matériaux sol-gel particulièrement préférés sont préparés essentiellement à partir de tétraméthoxysilane (TMOS) et de 3-aminopropyltriéthoxysilane (APTES) dans un rapport molaire TMOS/APTES de 1/0,01 à 1/0,30, de préférence de 1/0,01 à 1/0,15, avantageusement de 1/0,01 à 1/0,10, notamment de 1/0,02 à 1/0,06, tout particulièrement de 1/0,03 et comprennent donc des unités de l'un et l'autre(s) dans de telles proportions.

Pour rappel, un matériau sol-gel est un matériau obtenu par un procédé sol-gel consistant à utiliser comme précurseurs des alcoolates de formule M(OR)ₙ où M est un métal, notamment le silicium, et R un groupement alkyle, et à les hydrolyser. En présence d'eau, l'hydrolyse des groupements alkoxy (OR) intervient, formant des petites particules de taille généralement inférieure à 1 nanomètre. Ces particules s'agrègent et forment des amas qui restent en suspension sans précipiter, et forment un sol. L'augmentation des amas augmente la viscosité du milieu qui gélifie. Un matériau sol-gel est obtenu par séchage du gel, en évacuant le solvant en dehors du réseau polymérique formé.

Le matériau sol-gel de l'invention comprend des unités et est essentiellement préparé à partir de 2 à 4 polyalcoxysilanes, notamment de 2 ou 3 et particulièrement 2 polyalcoxysilanes. Le matériau final peut contenir de 50 à 95% de dérivés de polyalcoxysilanes.

Des composés structurants (polymères organiques, surfactants neutres, surfactants anioniques, surfactants cationiques, etc.), permettant d'obtenir une structure poreuse régulière et/ou des formes de cavité particulières, peuvent être ajoutés au sol de départ à condition qu'ils puissent être éliminés par lavage ou calcination sans détériorer les propriétés optiques et structurales de la matrice.

Le matériau sol-gel ci-dessus incorpore également une molécule sonde.

Qu'on le précise ou non, dans ce qui suit, le terme "matériau sol-gel" désigne aussi bien un matériau sol-gel seul qu'un matériau sol-gel incorporant une molécule sonde, sauf lorsque le contexte montre qu'il s'agit de l'un d'entre eux et pas de l'autre.

Une molécule sonde est une molécule adaptée aux composés ou polluants à piéger ou à détecter ou à piéger et détecter, avec lesquels elle peut réagir et peut être par exemple de la 4-amino-3-penten-2-one (Fluoral-P®) pour la détection du formaldéhyde. L'incorporation d'une molécule sonde permet d'élargir la gamme des composés ou polluants visés par l'invention.

Des molécules sondes préférées sont l'hydralazine pour la détection de l'acétaldéhyde, l'hexaldéhyde et le crotonaldéhyde ; la 2,4-dinitrophénylhydrazine pour la détection des aldéhydes et des cétones dans leur globalité ; la 4-amino-3-penten-2-one (Fluoral-P®) pour la détection du formaldéhyde, les oxydes d'iode (KlO₄, I₂O₄ et I₂O₅) pour les composés aromatiques ou les alcanes, un dérivé du triphénylméthane (bleu de bromothymol, bleu de bromophénol, vert de bromocrésol, rouge de crésol, phénolphtaléine, vert de malachite, etc.) ou un dérivé de l'azobenzène (hélianthine, rouge congo, rouge de méthyle, jaune de méthyle, jaune d'alizarine R, etc.) pour la détection des acides carboxyliques

Le pourcentage pondéral de molécules-sondes est avantageusement de 0,1 à 40%, de préférence de 10 à 40% et tout particulièrement de 10 à 30 % rapporté au poids total du matériau.

La présente demande a aussi pour objet un procédé de préparation d'un sol-gel ci-dessus caractérisé en ce que l'on mélange le(s) premier(s) polyalcoxysilane(s), de préférence le tétraméthoxysilane avec un de ses solvants organiques miscibles à l'eau, on ajoute ensuite le(s) second(s) polyalcoxysilane(s), on ajoute de l'eau, additionnée, si désiré, d'un catalyseur ou d'un agent structurant, ou des deux, et on continue l'agitation pour obtenir le sol puis le gel. Si désiré le sol est placé dans des moules pour obtenir des blocs du gel. Sous forme combinée, les molécules de tétraméthoxysilane et de second(s) polyalcoxysilane(s) sont dénommées "unité(s)".

Avantageusement on mélange à une température comprise entre -45 et +30°C le ou les premier(s) polyalcoxysilane(s) avec un de leurs solvants organiques miscibles à l'eau, l'on ajoute ensuite le ou les second(s) polyalcoxysilane(s), l'on ajoute de l'eau, additionnée, si désiré, d'un catalyseur ou d'un agent structurant, ou des deux, et de préférence seulement un agent structurant, et l'on continue l'agitation pour obtenir le sol puis le sol-gel attendu, selon une modalité avantageuse, on place le sol dans des moules pour obtenir des blocs du sol-gel attendu.

A noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que l'on ajoute un agent structurant, il s'agit de l'ajout d'un ou plusieurs agents structurants ou lorsque l'on dit que l'on incorpore une molécule-sonde, il s'agit d'une ou plusieurs molécules-sondes.

Dans des conditions préférentielles de mise en oeuvre du procédé ci-dessus décrit on mélange le(s) premier(s) polyalcoxysilane(s), de préférence le tétraméthoxysilane (TMOS) avec un de ses solvants organiques qui est notamment l'acétone, le formamide, la méthyléthylcétone, le chloroforme, le dichlorométhane, l'acide acétique, le méthanol, l'éthanol, le propanol, le butanol, le pentanol, l'hexanol et de préférence un alcool notamment un alcanol en C₁-C₅, avantageusement en C₁-C₃ et en particulier du méthanol.

Le mélange peut s'effectuer à une température comprise entre -45 et +30°C et de préférence entre -25 et -15°C, pendant une durée comprise entre 1 et 10 minutes et préférentiellement de 2 à 3 minutes. Avantageusement on effectue le mélange à une température comprise entre -25 et -15°C, pendant une durée comprise entre 2 et 3 minutes.

On ajoute ensuite le(s) second(s) polyalcoxysilane(s) et tout particulièrement le 3-(aminopropyl)triéthoxysilane, de préférence dans une proportion inférieure ou égale au premier. On continue l'agitation pour une durée généralement de 1 à 10 minutes et préférentiellement de 2 à 3 minutes. Pour finir, l'eau ajoutée est de préférence ultrapure, additionnée, selon les besoins, du catalyseur et/ou de l'agent structurant, et de préférence seulement d'un agent structurant. L'agitation est alors encore maintenue pour 10 à 120 secondes et plus particulièrement pour 40 à 60 secondes.

De préférence toutes les étapes de synthèse ci-dessus sont réalisées à basse température.

Le rapport molaire polyalcoxysilanes/solvant/eau est avantageusement de 1/4/1 à 1/100/30, particulièrement de 1/4/4 et tout particulièrement de 1/5/4.

On utilise avantageusement le polystyrène ou le polypropylène comme matériau constitutif des moules pour obtenir les blocs de gel attendu.

Dans d'autres conditions préférentielles de mise en oeuvre du procédé ci-dessus décrit, on procède en outre au séchage des blocs du sol-gel de façon à faire évaporer les solvants résiduels. Le séchage des matrices sol-gel se déroule avantageusement à une température contrôlée et sous atmosphère de gaz inerte sec (azote, argon, air, etc.). Le séchage des blocs du sol-gel peut notamment être réalisé en disposant un couvercle perméable aux gaz et plus particulièrement un film poreux à la surface des moules puis en plaçant ces moules dans une enceinte thermostatée à une température comprise entre 25 et 60°C et plus particulièrement à 45°C dans le cas d'un matériau n'incorporant pas de molécules-sonde et à 25°C dans le cas contraire. L'atmosphère de séchage sera préférentiellement un gaz inerte sec et pur (azote qualité U, air industriel qualité FID, etc.). La durée pour un séchage complet sera variable entre 2 heures et 10 jours et dans le cas préférentiel de 2 heures environ pour un bloc de volume d'environ 2 ou notamment 5.10⁻³ cm³.

Dans le cas où le matériau contient un ou plusieurs surfactants, ces derniers sont éliminés soit par lavage ou trempage dans une solution aqueuse ou organique ou encore par calcination, après gélification.
La présente demande a aussi pour objet un procédé de préparation d'un matériau sol-gel incorporant une molécule sonde capable de réagir sélectivement avec un composé-cible. L'incorporation de cette ou ces molécules-sonde peut se faire selon plusieurs méthodes.
- la méthode "one-pot", qui est la préférée, qui consiste à ajouter la molécule sonde directement lors de la préparation du sol. Dans ce cas, la molécule-sonde est directement encapsulée dans le réseau de silice. La dilution ou la dissolution des molécules-sonde peuvent se faire au choix dans le solvant ou dans l'eau servant à la préparation du sol. Le choix préférentiel sera de diluer ou de dissoudre la molécule-sonde dans le milieu où elle est la plus soluble ou la mieux miscible.

- La méthode par diffusion en voie gazeuse ou en voie liquide consiste à faire entrer la molécule-sonde dans les pores vides du matériau sol-gel après son séchage. Dans ce cas, les molécules-sonde sont adsorbées à la surface du matériau ou liées à cette surface par des liaisons non-covalentes (liaisons hydrogène ou liaisons ioniques). La méthode par diffusion en voie gazeuse consiste à mettre en contact les molécules-sonde sous forme gazeuse directement avec le matériau (sous vide partiel ou par circulation du gaz). La méthode par diffusion en voie liquide consiste à mettre le matériau sol-gel directement dans une solution (aqueuse ou solvant) contenant la molécule-sonde dissoute ou diluée.
- La méthode par fonctionnalisation ou post-dopage qui consiste à créer une liaison covalente entre le matériau sol-gel et la molécule-sonde. Pour cela, il est avantageux de fonctionnaliser la surface du matériau sol-gel pour améliorer sa compatibilité avec la molécule-sonde ou fonctionnaliser cette dernière.

Comme on l'a vu, on peut incorporer deux ou plusieurs molécules sondes.

Les matériaux sol-gel objet de la présente invention possèdent de très intéressantes propriétés et qualités. Ils sont doués notamment de remarquables propriétés de piégeage de toute une variété de solvants usuels et d'hydrocarbures aromatiques monocycliques et notamment le benzène, le toluène, les xylènes, le mésitylène et le styrène.

Ils sont également transparents dans l'UV, ce qui permet de mesurer directement l'absorbance des hydrocarbures aromatiques monocycliques piégés, ces hydrocarbures se distinguant généralement par leur absorbance UV.

Les matériaux sol-gel sont utilisables avec des hydrocarbures aromatiques monocycliques notamment sous forme gazeuse.

Plus généralement les matériaux sol-gel de l'invention peuvent piéger les composés qui absorbent dans l'UV-visible avec un coefficient d'extinction molaire supérieur à 250 M⁻¹cm⁻¹. La sensibilité est convenable pour des concentrations ≥1 ppmv et même d'une dizaine de ppbv pour les gaz qui ont un coefficient d'absorption élevé comme le styrène.

Ils permettent de réaliser la détection des BTEMXs directement par une mesure d'absorbance. Dans certains cas comme le cas du styrène, la métrologie du polluant peut aussi être réalisée via des mesures de fluorescence.

Utilisés sur une faible épaisseur, par exemple de 100 à 800 µm et avantageusement de 100 à 500 µm, ils permettent un piégeage réversible des HAMs et de leurs solvants usuels.

L'utilisation d'un spectrophotomètre, avantageusement miniaturisé et refroidi, permet la détection par mesure d'absorbance ou de fluorescence est d'excellente qualité et sensible.

Par rapport à des adsorbants poreux sous forme de cubes de silice, on peut noter en particulier les avantages suivants:
- le piégeage et la mesure sont possibles en une seule étape ;
- une seule chambre sert à l'exposition et à la mesure ;
- la désorption des gaz peut être effectuée sans chauffage par utilisation d'un système millifluidique adapté au capteur.

Grâce notamment à l'utilisation de deux groupes distincts de polyalcoxysilanes, premier(s) et second(s) polyalcoxysilanes tels que définis précédemment, par rapport à des adsorbants de benzène à base de matrices poreuses de polymères hybrides organique-inorganique à base d'alcoxydes de silicium (Si(OR)ₙR₄₋ₙ avec R=CH₃), on peut noter en particulier les avantages suivants:
- les étapes de synthèse et de séchage des capteurs poreux sont réduites à 4 heures au lieu de 2 mois;
- la taille des capteurs est réduite ainsi que leur coût de fabrication ;
- le nouveau matériau poreux permet une diffusion plus rapide des composés-cibles;
- le système millifluidique permet d'augmenter le rendement de piégeage ;
- le piégeage est réversible et les composés-cibles sont désorbés sans chauffage;
- le système de détection peut être miniaturisé et le rapport signal/bruit est amélioré.

Par ailleurs, les matériaux sol-gel objets de la présente invention permettent une longue autonomie de 300 à 400 mesures, ce qui correspondrait à 3 à 4 jours si une mesure est réalisée toutes les quinze minutes. Cette autonomie peut être augmentée à 6 à 8 jours si la cadence de mesure devient égale à 30 minutes et encore plus si la cadence de mesure diminue; en outre, des dispositifs de piégeage et/ou systèmes de détection les mettant en oeuvre sont robustes, de faible encombrement, portables et légers.

Les matériaux sol-gel objets de la présente invention ont pour propriété d'avoir un pH intrinsèque proche de 7. Grâce à l'utilisation des divers polyalcoxydes aminés précités, il est possible de faire évoluer le pH intrinsèque du matériau sans avoir recours à l'ajout d'une base (OH⁻). Les valeurs de pH se situent entre 7 et 8,2. Dans le cas préférentiel précité, le pH est égal à 7,5±0,3.

Lorsqu'elles incorporent une molécule sonde, les matrices peuvent piéger sélectivement toute une variété de composés grâce à une réaction spécifique entre la molécule-sonde et le composé-cible. Dans ce cas, le piégeage de l'analyte (composé-cible) est irréversible. On peut alors mesurer quantitativement le composé-cible piégé en détectant le produit issu de l'interaction ou de la réaction de celui-ci avec la molécule sonde. Les autres composés-cibles présents dans le milieu au moment de l'analyse peuvent aussi être piégés dans ce cas mais ne sont pas détectés en l'absence de réaction spécifique. Dans d'autres cas, les molécules-sonde sont sensibles à de faibles variations de pH dues au piégeage d'un composé-cible acide, qui interagit via des liaisons faibles avec la molécule-sonde. La réaction est dans ce cas réversible.

Ces propriétés sont illustrées ci-après dans la partie expérimentale. Elles justifient l'utilisation des matériaux sols gels ci-dessus décrits, ainsi que des matériaux sols gels incorporant une molécule sonde, dans le piégeage d'hydrocarbures aromatiques monocycliques, ainsi que d'autres composés-cibles et/ou dans leur détection.

C'est pourquoi la présente demande décrit un procédé de piégeage d'hydrocarbures aromatiques monocycliques ou d'autres composés d'intérêt utilisant comme capteur un matériau sol-gel ci-dessus décrit, dans lequel on met en contact un flux susceptible de contenir des hydrocarbures aromatiques monocycliques ou d'autres molécules-cible avec un matériau sol-gel ci-dessus décrit, éventuellement incorporant une molécule sonde, ou fait circuler un tel flux sur celui-ci.

Le flux susceptible de contenir des hydrocarbures aromatiques monocycliques peut provenir d'une atmosphère polluée. Il peut circuler à un débit de 10 mL.min⁻¹ à 1,1 L.min⁻¹.

La présente demande a aussi pour objet un procédé de préparation d'un matériau sol-gel incorporant une molécule sonde ci-dessus, caractérisé en ce que l'on ajoute au matériau sol-gel une molécule sonde soit directement lors de la préparation du sol du matériau sol-gel soit par diffusion de cette molécule-sonde dans ledit matériau par diffusion par voie gazeuse ou par voie liquide.

La présente demande a aussi pour objet un procédé de préparation d'un matériau sol-gel incorporant une ou plusieurs molécules-sonde ci-dessus citée(s), caractérisé en ce que l'on ajoute au matériau sol-gel, à basse température de -45 à +15 °C la ou les molécule(s)-sonde soit directement lors de la préparation du sol du matériau sol-gel soit par diffusion de cette ou ces molécule(s)-sonde(s) dans ledit matériau par diffusion par voie gazeuse ou par voie liquide. Le matériau sol-gel a un pH intrinsèque proche de 7, que l'on peut faire varier dans une gamme de pH de 7 à 8,2, et par exemple dans le cas où l'unité de premier alcoxysilane est le TMOS et l'unité de second polyalcoxysilane est l'APTES, en augmentant la proportion de l'APTES par rapport au TMOS.

En plus de piéger les hydrocarbures aromatiques monocycliques, on peut également les détecter et/ou les doser. A cette fin, on peut procéder également à leur piégeage.

La présente demande décrit donc également un procédé ci-dessus dans lequel en outre, on procède à la détection des hydrocarbures aromatiques monocycliques et de leurs solvants usuels piégés dans les matériaux sol-gel ci-dessus, éventuellement incorporant une molécule sonde.

La détection peut être réalisée notamment par mesures optique, massique, ou acoustique.

Lorsque la détection est réalisée par mesure optique, on choisit de préférence la longueur d'onde pour laquelle l'absorbance du composé-cible est la plus élevée. On peut aussi choisir la longueur d'onde pour laquelle la fluorescence du composé-cible est la plus élevée.

Pour doser les hydrocarbures aromatiques monocycliques piégés sur les matériaux sol-gel ci-dessus, éventuellement incorporant une molécule sonde, on peut notamment effectuer une mesure de la variation d'absorbance, de fluorescence, de luminescence, de masse ou de fréquence de résonnance du monolithe lorsqu'il est exposé à un flux contenant le (ou les) composé-cible. La mesure obtenue comparée à la mesure obtenue à partir de flux calibrés de composé-cible donne directement une information sur la quantité et/ou la nature du composé-cible contenu dans le flux d'exposition.

Comme on l'a vu ci-dessus, lorsqu'ils incorporent une molécule sonde, les matériaux sol-gel ci-dessus peuvent piéger toute une variété de composés-cibles comme les composés aromatiques monocycliques, les aldéhydes, les alcanes, les acides carboxyliques, les cétones et le chlore, notamment les composés aromatiques monocycliques, les aldéhydes, les alcanes, les acides carboxyliques et les cétones.

Le flux peut correspondre à un gaz susceptible de contenir ces composés-cibles qui peuvent provenir d'une atmosphère polluée. Le flux gazeux peut circuler à un débit allant de 10 mL.min⁻¹ à 1,1 L.min⁻¹.

En plus de piéger ces composés-cibles, on peut également les détecter et/ou les doser. A cette fin, on peut procéder également à leur piégeage.

La présente demande a donc également pour objet un procédé ci-dessus dans lequel en outre, on procède à la détection des composés-cibles piégés sur les matériaux sol-gel ci-dessus incorporant une molécule sonde.

La détection peut être réalisée notamment par mesures optique, massique, ou acoustique.

Lorsque la détection est réalisée par mesure optique, on choisit de préférence la longueur d'onde pour laquelle l'absorbance, la fluorescence ou la luminescence de la molécule-sonde, ou du produit formé par l'interaction ou la réaction entre la molécule-sonde et les composés cibles est la plus élevée.

Pour doser les composés-cibles piégés sur les matériaux sol-gel ci-dessus incorporant une molécule sonde, on peut notamment mesurer au choix les variations d'absorbance ou de fluorescence ou de luminescence de la molécule-sonde au cours du temps ou les variations d'absorbance ou de fluorescence ou de luminescence du produit de la réaction entre la molécule-sonde et le polluant-cible au cours du temps. Dans les deux cas les résultats seront comparés à des résultats obtenus dans les mêmes conditions d'exposition avec des flux calibrés de composés-cibles.

La présente demande a également pour objet un système de piégeage d'hydrocarbures aromatiques monocycliques ou d'un composé-cible ci-dessus utilisant comme capteur un matériau sol-gel ci-dessus décrit, éventuellement incorporant une molécule sonde. Un tel capteur comprend habituellement un bloc de matériau sol-gel ci-dessus décrit de forme reproductible, par exemple parallélépipédique, cylindrique, cubique, trapézoïdale, etc., de surface inférieure ou égale à 150 mm², et de préférence inférieure ou égale à 100 mm², et d'épaisseur inférieure ou égale à 2 mm. Ce capteur est habituellement utilisé dans une cellule comprenant essentiellement :
- un système millifluidique pour l'écoulement gazeux et dans lequel est inséré un matériau sol-gel ci-dessus décrit ;
- une chambre d'exposition munie de fenêtres optiques et d'ouvertures pour le système millifluidique (entrée et sortie de gaz) ;

Dans le présent exposé, le terme « système millifluidique» désigne un système permettant le passage d'un flux gazeux au débit de 10 mL.min⁻¹ à 1,1 L.min⁻¹, de préférence de 100 mL.min⁻¹ pour l'étape de piégeage.

La présente demande a tout autant pour objet un système de détection d'hydrocarbures aromatiques monocycliques et leurs solvants usuels utilisant comme capteur un matériau sol-gel ci-dessus décrit, éventuellement incorporant une molécule sonde. Un tel système de détection comprend habituellement
- une chambre d'exposition ci-dessus ;
- un système millifluidique ci-dessus ;
- un système optique de collimation et focalisation de la lumière d'analyse ;
- des fibres optiques pour véhiculer la lumière ;
- une (micro)pompe adaptée au débit désiré ;
- un spectrophotomètre ou autre dispositif de détection.

Le système de détection peut être utilisé notamment comme suit :
Le bloc de matériau sol-gel est inséré dans le système millifluidique. Ce dernier est placé dans la chambre d'exposition. On fait circuler le mélange de composés-cibles gazeux dans le système millifluidique pendant un temps court (15 sec à 2 min). Pendant la durée de l'exposition, le spectre d'absorption du capteur est collecté toutes les secondes de la manière décrite ci-après. La lumière d'analyse provenant d'une lampe UV (deutérium) est véhiculée à l'aide d'une fibre optique vient éclairer en continu la fenêtre d'entrée de la chambre. Le faisceau de lumière, collimaté à l'aide d'une lentille (focale=10 mm) et d'un connecteur SMA placé sur la fenêtre d'entrée, vient éclairer le capteur sur une petite surface, par exemple 1 mm². La lumière transmise est collectée dans le même axe via une seconde lentille et un second connecteur SMA placé sur la fenêtre de sortie de la chambre. Le faisceau de lumière transmis est véhiculé à l'aide d'une fibre optique vers un spectrophotomètre éventuellement miniature. Un spectre d'absorption du capteur est collecté toutes les secondes, chaque acquisition durant de 8 à 1000 msec (préférentiellement 20 msec).

Lorsque l'acquisition est terminée, on peut libérer les composés-cibles piégés et purger le capteur. On peut à cette fin exposer le capteur à un flux d'air de 4 L.min⁻¹ durant environ 5 minutes.

Le signal d'absorbance du capteur exposé à chaque composé-cible est acquis sur une large gamme de concentration du composé-cible et sur une large gamme d'humidité relative du mélange gazeux. A partir de ces données, des courbes de calibration sont établies pour chaque composé-cible en fonction de sa concentration et de l'humidité du mélange gazeux. Ces courbes de calibration sont stockées dans une banque de données qui servira à la déconvolution spectrale d'un spectre du capteur exposé à un mélange inconnu de composés-cibles sous forme gazeuse.

Le signal d'absorbance est déconvolué à partir d'une banque de données des spectres d'absorption de chacun des composés-cibles déterminés auparavant dans une matrice telle que décrite précédemment.

Pour l'étape de relargage, le débit du flux gazeux peut varier de 2 L.min⁻¹ à 5 L.min⁻¹ et est fixé de préférence à 4 L.min⁻¹.

Dans le cas d'une matrice dopée de molécules-sonde aptes à réagir avec les composés-cibles, la réaction entre la molécule-sonde et le composé-cible est dans la majorité des cas irréversible. L'étape de purge n'est pas réalisée pour réaliser un relargage mais pour purger le circuit fluidique des composés présents dans le mélange gazeux et n'ayant pas réagi avec les molécules-sonde. Dans ce cas, le débit du flux de purge sera avantageusement fixé à une valeur de 10 mL.min⁻¹ à 1,1 L.min⁻¹.

Dans le cas d'un matériau sol-gel contenant une ou plusieurs molécules-sonde dérivées soit du triphénylméthane soit de l'azobenzène, la purge s'effectue avantageusement en modifiant les conditions de pH du flux ou du matériau sol-gel jusqu'à observer un retour aux conditions de mesures optiques initiales.

Par ailleurs, lorsque le circuit fluidique fonctionne en continu (ou en boucle) grâce à une pompe à circulation pendant une durée suffisamment longue (quelques secondes à 1 heure), pendant laquelle les composés-cibles entrent dans la matrice et réagissent avec les molécules-sonde, les mesures optiques sont réalisées (collecte de spectres d'absorption ou de fluorescence) à intervalles de préférence réguliers (1 à 300 secondes), pendant cette durée.

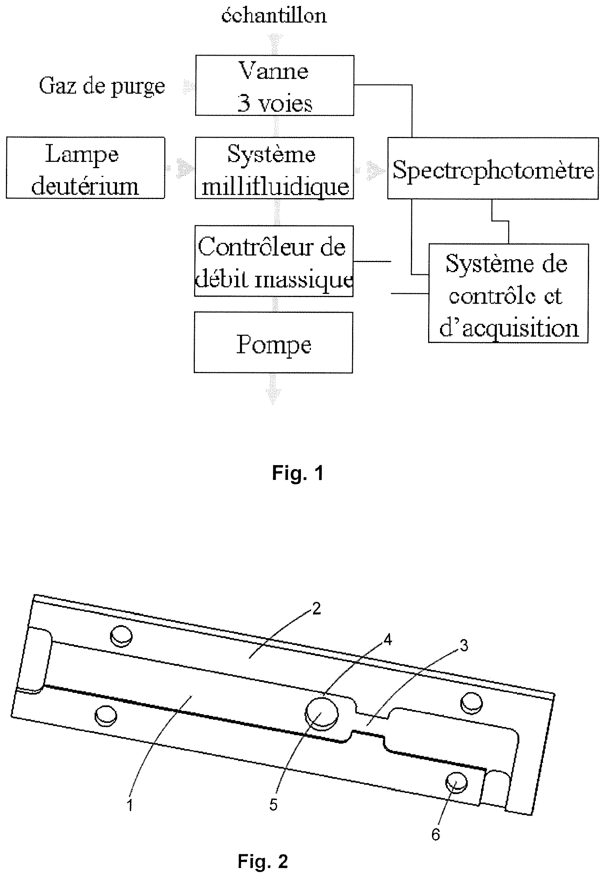

Les exemples qui suivent illustrent la présente demande et l'invention sera mieux comprise si l'on se réfère aux dessins annexés sur lesquels
- la figure 1 représente une vue schématique du dispositif de mesure dans sa totalité (cellule de mesure, spectrophotomètre, lampe deutérium, pompe) ;
- la figure 2 représente une vue en perspective d'une des plaques de cuivre constituant le système millifluidique ;

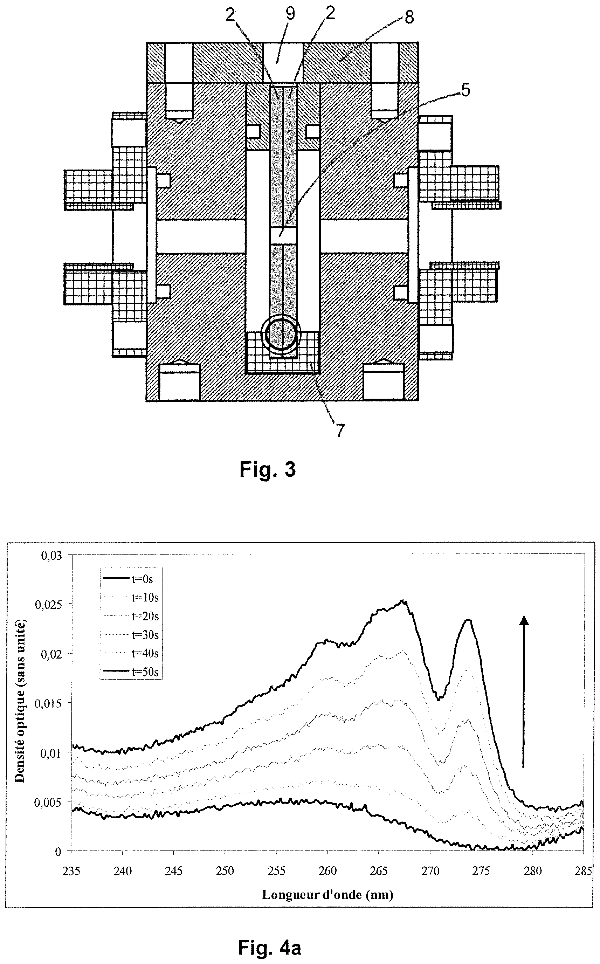

- la figure 3 représente une vue en coupe transversale d'une chambre d'exposition ;

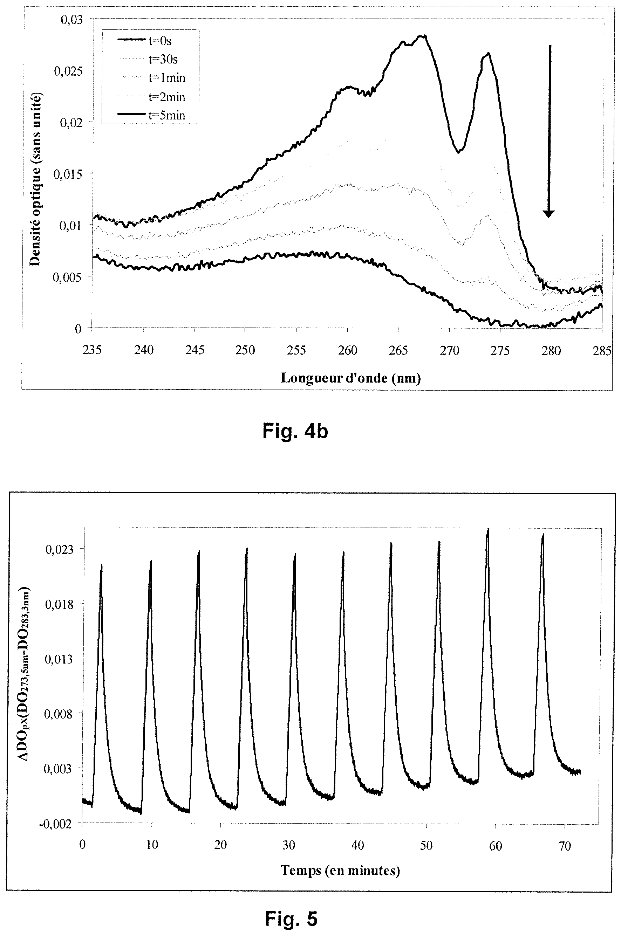

- la figure 4 représente les résultats d'absorbance du p-xylène en fonction de la longueur d'onde; pour un capteur exposé à un flux d'azote contenant 11,86 ppmv de p-xylène, où la figure 4a représente l'évolution des spectres d'absorption au cours du piégeage d'un composé-cible et la figure 4b représente l'évolution du spectre d'absorption d'une matrice au cours du relargage (purge de la matrice dans le cas d'un piégeage simple du composé-cible par adsorption physique) ;
- la figure 5 représente une série d'expositions d'un bloc de sol-gel à des mélanges gazeux suivie de la purge du bloc de sol-gel. Chaque "impulsion" correspond à une augmentation linéaire d'absorbance pendant une minute d'exposition à un composé-cible, puis à une diminution exponentielle de l'absorbance pendant 5 minutes lors de la purge. La valeur de la pente du signal de croissance de l'absorbance est caractéristique de la concentration du composé-cible dans le flux d'exposition ;

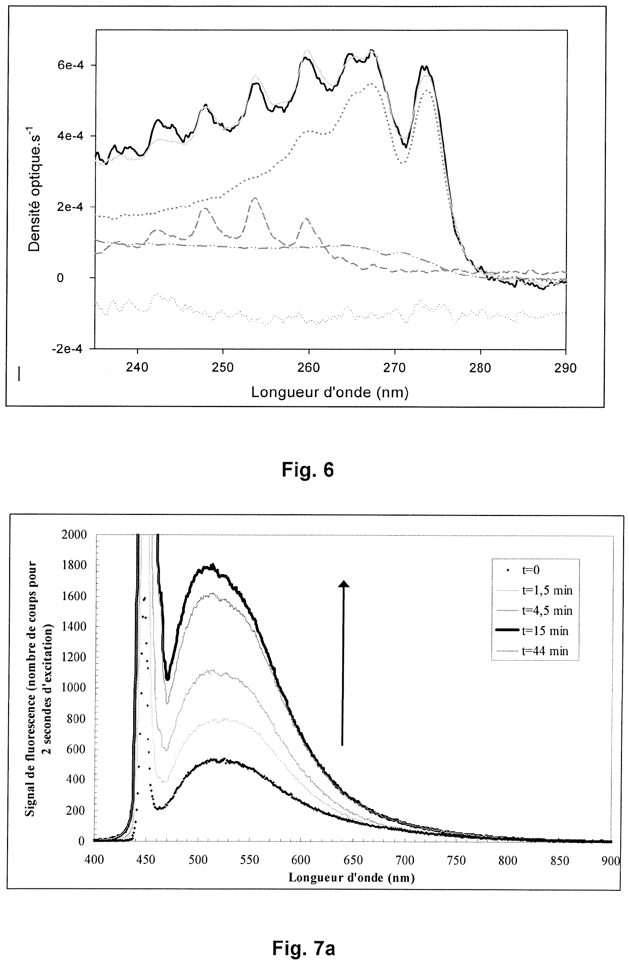

- la figure 6 représente un spectre enregistré lors d'une exposition à un mélange de composés-cibles (benzène, para-xylène et mésitylène). Le spectre correspond à la somme des spectres des composés-cibles pris individuellement dans les mêmes conditions ;
- la figure 7a représente les spectres de fluorescence enregistrés lors de l'exposition d'un bloc de sol-gel dopé de molécules-sonde (ici le Fluoral-P®) à un flux gazeux de 200 mL.min-1 contenant 10 ppbV de formaldéhyde. Les variations spectrales au cours du temps correspondent à une augmentation de la fluorescence du produit issu de la réaction entre le Fluoral-P® et le formaldéhyde (longueur d'onde d'excitation = 405nm - durée d'excitation = 2 secondes).

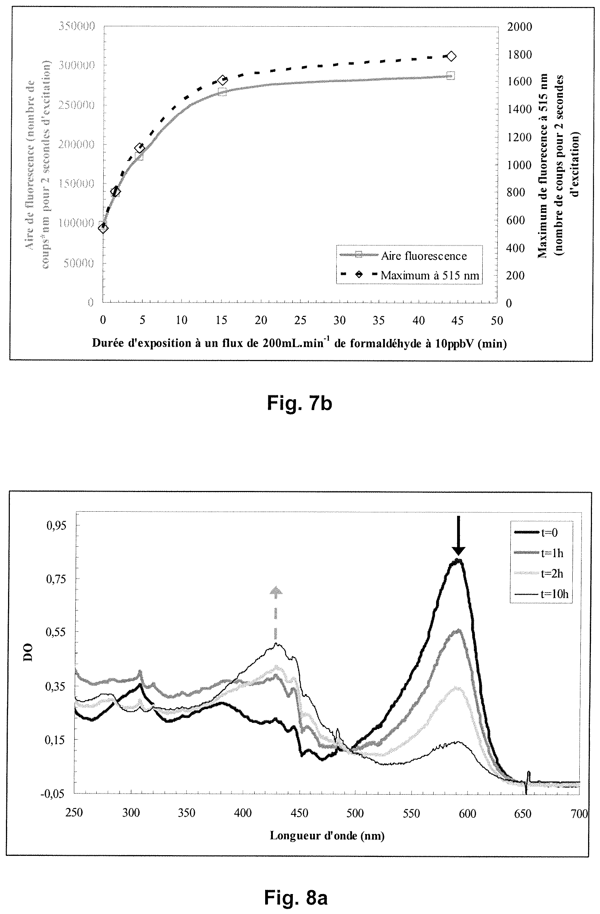

- la figure 7b représente les évolutions de l'aire de fluorescence (entre 470 et 750 nm) d'une part (courbe trait plein) et du maximum de fluorescence à 520 nm (courbe pointillés) d'autre part, au cours de l'exposition d'une matrice dopée au Fluoral-P® à un flux de 200mL.min-1 contenant 10 ppbv de formaldéhyde. La pente à l'origine de ces 2 courbes est caractéristique de la concentration en formaldéhyde du flux d'exposition.
- la figure 8a représente les spectres d'absorption pris lors de l'exposition d'un bloc de sol-gel dopé de molécules-sonde (ici le bleu de bromophénol) à une atmosphère contenant de l'acide acétique à la pression de vapeur saturante. Les variations spectrales correspondant d'une part à une disparition du réactif (bleu de bromophénol, diminution de l'intensité de la bande d'absorption à 592nm) et d'autre part à une formation du produit de réaction (apparition d'une bande d'absorption à 431 nm).

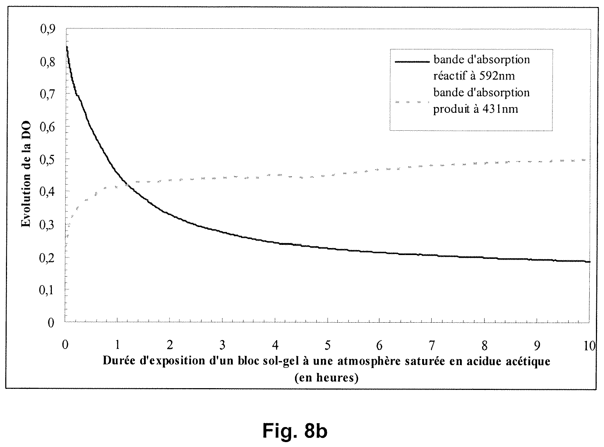

- la figure 8b représente les évolutions de densité optique correspondant d'une part à la disparition du réactif, le bleu de bromophénol (courbe trait plein) et à l'apparition du produit de la réaction (bleu de bromothymol protoné) d'autre part lors de l'exposition d'un bloc dopé avec du bleu de bromophénol à une atmosphère contenant de l'acide acétique à la pression de vapeur saturante. La pente à l'origine de ces 2 courbes est caractéristique de la concentration en acide acétique du flux d'exposition. Cette réaction est réversible par lavage du monolithe et désorption de l'acide acétique.

### Exemple 1 : Préparation d'un bloc de sol-gel APTES-TMOS

### Stade 1 :

On chauffe à l'étuve à 50°C pendant 24 heures des plaques multipuits de 96 puits en polystyrène (Greiner Bio-one, Elisa-microplates flat bottom, réf. 655001) en dégazant l'étuve à trois reprises pendant cette période. Cette étape permet de dégazer les moules en polystyrène pour minimiser le relargage du styrène monomère qui pourrait par la suite venir se piéger dans le matériau sol-gel au cours de l'étape de séchage du gel.

On mélange à l'agitateur magnétique 3,4 mL de tétraméthoxysilane (TMOS, Fluka, réf. 87680) et 4,8 mL de méthanol (Fluka, réf. 65540) pendant 2 minutes dans un bêcher en pyrex placé dans un bain à - 25°C. On ajoute ensuite 0,2 mL de 3-aminopropyl)triéthoxysilane (APTES, Fluka, réf. 09324) au mélange à l'aide d'une micropipette. Après 2 minutes d'agitation supplémentaires, 1,7 mL d'eau ultrapure Millipore est ajoutée. On agite encore 30 secondes. On obtient un sol utilisé tel quel au stade suivant.

### Stade 2:

En maintenant le sol à -25±5 °C et en procédant le plus rapidement possible, à l'aide d'une micropipette, on place 38 µL de sol dans chacun des puits d'une plaque multipuits référence Greiner Bio-one Elisa-microplates flat bottom (655101).

### Stade 3:

Une fois que le sol est gélifié, on recouvre la plaque multipuits d'un film perméable aux gaz (Gas permeable adhesive seals, ABGene, réf. AB-0718). On place la plaque à l'étuve à 40°C pour une durée de 2 heures. Puis on sort les plaques de l'étuve et on démoule les matrices que l'on place dans des boîtes hermétiques en polypropylène. On remet les boîtes dans l'étuve à 40°C pour 4 heures afin de terminer le séchage.

Après séchage, on obtient des blocs de sol-gel ayant la forme de disques monolithiques. Le diamètre moyen des blocs de sol-gel obtenus est de 3,6 mm et leur épaisseur de 200±25 µm environ. Leur surface spécifique moyenne = 750 m².g⁻¹. Celle-ci a été évaluée en établissant des isothermes d'adsorption et de désorption de l'azote à la température de l'azote liquide et en analysant les isothermes à l'aide de divers modèles analytiques proposés dans la littérature tels que le modèle BET (Brunauer, Emmet et Taylor).

Leur volume poreux moyen est 0,67 cm³.g⁻¹. Celui-ci a été évalué en établissant des isothermes d'adsorption et de désorption de l'azote à la température de l'azote liquide et en analysant les isothermes à l'aide du modèle analytique DFT (Théorie de la Fonctionnelle de Densité).

La distribution des tailles de pores a été évaluée par la méthode DFT (Théorie de la Fonctionnelle de Densité) basée sur des méthodes de calcul du potentiel d'interaction entre les molécules d'adsorbat et entre ces dernières et la surface des pores, qui permet de reconstituer une donnée macroscopique telle que l'isotherme d'adsorption à partir des données microscopiques tels que les potentiels d'interaction. Le modèle de formes de pores ("mélange sphériques et cylindriques") a été utilisé pour le calcul.

Les micropores sont définis comme des pores ayant des diamètres ≤ 20 Å.

Les mésopores sont définis comme des pores ayant des diamètres 20 ≤ d ≤ 500 Å.

Les résultats obtenus pour le matériau de l'exemple 1, sont les suivants :
- Répartition micropores/mésopores (en surface) = 35/65 %
- Répartition micropores/mésopores (en volume) = 15/85 %

### Exemple 2 : Préparation d'un bloc de sol-gel APTES-TMOS

Différentes formes et tailles de matrices ont été réalisées :
- parallélépipédique (Star-Pack, réf. 47304 et réf. 271512 et réf. 303 - Evergreen, réf. 201-3111-010)
- cylindrique (Spex industries Inc., réf. 3111 - Greiner Bio-one Elisa-microplates flat bottom, réf. 655001)
- trapézoïdale (Agar scientific, réf. G3533)

On a préparé des blocs de sol-gel APTES-TMOS comme à l'exemple 1 en utilisant les moules cylindriques Greiner Bio-one Elisa-microplates flat bottom (réf. : 655101) et on a obtenu des blocs de diamètre 3,6 mm et d'épaisseurs variables entre 80 et 1000 µm (en fonction du volume de sol de départ).

Pour chacun des autres moules référencés ci-dessus et dans le cas d'un matériau tel que celui de l'exemple 1, le bloc Sol-Gel final (c'est-à-dire après séchage) conserve la forme du moule mais son volume est environ 8 fois plus faible que le volume de départ. Il y a un facteur de rétrécissement égal à 2 dans les 3 dimensions en raison de l'expulsion des solvants résiduels au cours de l'étape de séchage.

### Exemple 3 : Préparation d'un bloc de sol-gel APTES-TMOS incorporant une molécule sonde

On a préparé des blocs de sol-gel APTES-TMOS incorporant différentes molécules sonde : 4-amino-3-penten-2-one (TCI, réf. A5350) ; bleu de bromophénol (Sigma-Aldrich, réf. 114391) ; rouge de méthyle (Sigma-Aldrich, réf. 250198) ; hélianthine (Sigma-Aldrich, réf. 114510) ; rouge Congo (Aldrich, réf. 860956) ; vert de bromocrésol (Sigma-Aldrich, réf. 114359) et pourpre de bromocrésol (Sigma-Aldrich, réf. 114375).

L'incorporation de la 4-amino-3-penten-2-one a été faite selon trois procédés différents décrits ci-dessous :
- Dopage "one-pot" : on procède comme à l'exemple 1 mais le méthanol est remplacé par un mélange méthanol + 4-amino-3-penten-2-one. Différentes concentrations ont été utilisées (100, 300, 500 et 750 mg de 4-amino-3-penten-2-one dans 4,8 mL de méthanol). Pour le reste du protocole on procède exactement comme à l'exemple 1 sauf qu'ici le séchage des blocs de sol-gel se fait à 25°C.
- "Post-dopage liquide" : des blocs de sol-gel tels que ceux obtenus à l'exemple 2, sont plongés dans une solution aqueuse de concentration en 4-amino-3-penten-2-one égale à 2.10⁻³ mol.L⁻¹ pendant 2 heures puis séchés sous gaz inerte.
- "Post-dopage gazeux" : des blocs de sol-gel tels que ceux obtenus à l'exemple 2 sont mis dans une enceinte sous une pression réduite de 133,3 Pa (1 torr) en présence de 4-amino-3-penten-2-one en poudre. L'enceinte est chauffée à 40 °C pendant 15 heures, pendant lesquelles la 4-amino-3-penten-2-one se sublime et pénètre dans les blocs de sol-gel, plus précisément dans les pores des blocs de sol-gel. A la fin de l'exposition, de l'azote sec est introduit dans l'enceinte pour rétablir la pression atmosphérique et récupérer les blocs de sol-gel dopés.

L'incorporation des autres molécules-sondes précédemment citées (bleu de bromophénol ; rouge de méthyle ; hélianthine ; rouge Congo ; vert de bromocrésol et pourpre de bromocrésol) a été réalisée uniquement par la méthode de dopage "one-pot".

On procède comme à l'exemple 1 mais on remplace le méthanol par un mélange de méthanol et de l'un des composés précédemment cités (bleu de bromophénol ; rouge de méthyle ; hélianthine ; rouge Congo ; vert de bromocrésol et pourpre de bromocrésol). Différentes concentrations ont été utilisées, préparées à partir de 0,5 ; 1 ; 10 ; 30 ; 50 et 100 mg dissouts dans 4,8 mL de méthanol. Pour le reste du protocole, on procède exactement comme à l'exemple 1 sauf que le séchage des blocs de sol-gel se fait dans une enceinte thermostatée à 25°C.

### Exemple 4 : Préparation d'un système millifluidique

On a réalisé un système millifluidique constitué de deux plaques planes de cuivre accolées. Le circuit macro-fluidique 1 de 3,5 mm de largeur, 36 mm de longueur et de 0,5 mm de profondeur a été creusé dans chaque plaque de cuivre 2 et lors du montage les deux parties évidées sont accolées. Le circuit millifluidique 1 est en forme de L et comporte un étranglement 3 dans la branche la plus longue de sorte que la largeur du circuit n'y est que de 1 mm (Fig.2). On a installe un bloc de sol-gel de l'exemple 2 et dans un autre système un bloc de sol-gel de l'exemple 3 juste avant l'étranglement 3 au niveau de 4. L'étranglement du circuit permet le bon maintien du disque de sol-gel. Un percement 5 des plaques est prévu au niveau de l'emplacement central du bloc de sol-gel pour le passage du faisceau de lumière d'analyse. D'autres percements 6 ont été prévus pour serrer ensemble les plaques 2.

On a aussi réalisé des systèmes millifluidiques avec deux plaques 2 de PTFE, avec deux plaques 2 en cuivre et avec deux plaques 2 en acier inoxydable.

### Exemple 5 : Préparation d'un dispositif de mesure

L'ensemble de l'exemple 4 en cuivre a été inséré dans une chambre de mesure en PTFE équipé de deux fenêtres optiques en quartz (Fig.3).

La chambre d'exposition comprend le système millifluidique. Ce dernier est maintenu fixe dans la chambre d'exposition grâce à une embase 7 prévue à cet effet et à un couvercle 8. En plus du maintien du système millifluidique, le couvercle est percé en son centre 9 pour le passage du mélange gazeux.

Pour l'analyse optique du capteur, le trajet optique du faisceau d'analyse est perpendiculaire au système millifluidique et passe au centre du capteur constitué par un bloc de sol-gel de l'exemple 2 respectivement de l'exemple 3. Des connecteurs standard pour fibres (de type SMA) et des lentilles (focale=10 mm) placés à l'entrée et la sortie optiques de la chambre d'exposition permettent une collimation du faisceau lumineux véhiculé par des fibres optiques à l'entrée et à la sortie de la chambre d'exposition. A la sortie de la chambre d'exposition, le faisceau de lumière transmise est amené à un spectrophotomètre (Ocean Optics, QE6500). Un spectre d'absorption du capteur est collecté avant son exposition au flux de produit testé. Dès la mise sous flux du système millifluidique et ce pendant une durée pouvant varier entre 30 secondes et 2 minutes dans le cas d'un bloc de l'exemple 2 et entre 1 et 120 minutes dans le cas d'un bloc tel que ceux de l'exemple 3, un spectre d'absorption est collecté toutes les secondes et les données sont stockées pour un traitement ultérieur.

### Exemple 6 : Piégeage et de dépiégeage d'un composé-cible

Un exemple de mesure de l'exposition d'un capteur à un flux d'azote contenant 11,86 ppmv de p-xylène est montré dans la figure 5.

On a introduit un bloc de sol-gel APTES-TMOS obtenu à l'exemple 2 dans le système millifluidique de l'exemple 4 et le système de l'exemple 4 a été inséré dans dispositif de mesure de l'exemple 5.

On a préparé un mélange gazeux contenant 11,86 ppmv de para-xylène dilué dans de l'azote.

Ce mélange gazeux a été envoyé au débit de100 mL.min⁻¹ dans le système ci-dessus pendant une minute. Puis on a fait passer un flux d'air ambiant dans le circuit millifluidique à un débit de 4 L.min⁻¹ pendant 5 minutes.

Dès l'introduction du flux d'azote contenant le p-xylène dans le circuit millifluidique les spectres ont été collectés toutes les secondes. La figure 4a montre la montée du signal qui correspond à l'absorbance du p-xylène piégé, mesurée sur une gamme de longueurs d'onde allant de 235 à 285 nm. On voit sur la figure 4b la diminution de l'absorbance du p-xylène jusqu'à atteindre une valeur correspondant à celle mesurée avant l'exposition au p-xylène.

On a aussi réalisé le même type d'expériences pour des concentrations en gaz différentes (allant de 0 à 120 ppmv) et pour différents types de gaz (toluène, benzène, p-xylène, mésitylène) et dans différentes conditions d'humidité relative (0 à 94%).

### Exemple d'application 1 : Mesure d'un composé-cible sans humidité

On a procédé au dosage de p-xylène par mesure d'absorbance comme suit :
Préalablement au dosage du p-xylène, on a créé une banque de données des spectres d'absorption du p-xylène dans le capteur de l'exemple 2.

On a préparé un flux gazeux contenant 11,86 ppmv de para-xylène dilué dans de l'azote. Le débit d'exposition a été fixé à 100 mL.min⁻¹.

L'enregistrement d'une mesure s'est déroulé en accord avec le chronogramme suivant :
- Mise en place du bloc de sol-gel de l'exemple 2 dans l'ensemble de l'exemple 4 lui-même placé dans le dispositif de l'exemple 5;
- Purge du dispositif et stabilisation de la lampe et du spectrophotomètre (Ocean Optics, QE65000) pendant 10 minutes ;
- Enregistrement du spectre d'absorption du bloc de sol-gel ;
- Basculement de la vanne d'entrée de gaz pour permettre l'exposition du bloc de sol-gel au flux gazeux en enregistrant un spectre par seconde pendant une minute ;
- Basculement de la vanne pour effectuer une purge pendant 5 minutes avec de l'air.

Chaque cycle de mesure a duré 6 minutes en moyenne.

Le taux d'humidité était de 0%.

Les résultats obtenus sont reportés sur la figure 5.

On observe que la réponse du capteur est bien reproductible. La mesure de la montée du signal en fonction du temps (pente de la courbe) donne des valeurs répétables d'un cycle à l'autre avec un écart type très faible.

### Exemple d'application 2 : Mesure d'un composé-cible en présence d'humidité

On a procédé comme à l'exemple d'application 1 mais le taux d'humidité relative du flux d'exposition était de 80%.

Les résultats obtenus sont reportés sur la figure 6.

On observe que la réponse du capteur est bien reproductible même avec ce taux élevé d'humidité du mélange. La mesure de la montée du signal en fonction du temps (pente de la courbe) donne des valeurs répétables d'un cycle à l'autre avec un écart type faible de 6,3%.

Le capteur a une réponse reproductible dans le domaine d'humidité relative exploré de 0 à 94%.

### Exemple d'application 3 : Mesure d'un mélange de composés-cibles sans humidité

On a procédé comme à l'exemple d'application 1 mais en utilisant un mélange contenant 19,9 ppmv de benzène, 7,4 ppmv de mésitylène et 18,3 ppmv de p-xylène.

Les résultats obtenus sont reportés sur la figure 7.

Le signal d'absorbance est déconvolué à partir d'une banque de données des spectres d'absorption des différents composés-cibles dans ce dispositif.

On observe que les teneurs de composés-cibles extraites de la déconvolution spectrale du spectre du mélange des composés-cibles sont très proches de celles dans le mélange. En effet on obtient par la déconvolution une teneur de 19,6 ppmv de benzène, 7,3 ppmv de mésitylène et 17,2 ppmv de p-xylène.

On en conclut que le capteur capte bien tous les hydrocarbures monocycliques, indépendants ou en mélange, avec la même efficacité que lorsque chacun des composés-cibles est piégé séparément.

### Exemple d'application 4 : Détection d'un composé-cible avec une matrice dopée à la 4-amino-3-penten-2-one par mesure de fluorescence.

On a procédé au dosage du formaldéhyde par mesure de fluorescence comme suit :
Préalablement au dosage du formaldéhyde, on a crée une banque de données des spectres de fluorescence dans un capteur de l'exemple 3 dopé avec de la 4-amino-3-penten-2-one (Fluoral-P®) par la méthode "one-pot" (500mg dans 4,8 mL de méthanol).

On a préparé un flux gazeux contenant 10 ppbv de formaldéhyde dilué dans de l'air sec (qualité FID, Messer, réf. 27880). Le débit d'exposition a été fixé à 200 mL.min⁻¹.

L'enregistrement des mesures s'est déroulé en accord avec le chronogramme suivant :
- mise en place du bloc de sol-gel de l'exemple 3 dans le système millifluidique de l'exemple 4 et insertion de cet ensemble dans le dispositif de mesure de l'exemple 5 ;
- purge du dispositif et stabilisation de la lampe d'excitation et du spectrophotomètre (Ocean Optics, réf. QE65000) pendant 15 minutes ;
- enregistrement du spectre de fluorescence du bloc de sol-gel ;
- basculement de la vanne d'entrée pour permettre l'exposition du bloc de sol-gel au flux gazeux et enregistrement d'un spectre de fluorescence toutes les 30 secondes (excitation préalable pendant 2 secondes) pendant 45 minutes.

Les résultats sont reportés sur les figures 7a et 7b.

### Exemple d'application 5 : Détection d'un composé-cible avec une matrice dopée au bleu de bromophénol par mesure d'absorbance.

L'expérience a consisté à exposer un bloc de sol-gel de l'exemple 3 dopé avec du bleu de bromophénol par la méthode "one-pot" (0,25 mg dans 4,8 mL de méthanol) dans une atmosphère saturée en acide acétique (pression de vapeur saturante dans les conditions standard de température et de pression). Le bleu de bromophénol réagit avec l'acide acétique qui a diffusé à l'intérieur de la matrice pour former un produit dont le spectre d'absorption diffère de celui du réactif de départ (voir figure 8a). On a mesuré dans ce cas, la montée du signal correspondant à l'absorption du produit formé au cours de la réaction (bande d'absorption à 431 nm) en fonction de la durée d'exposition, et la décroissance du signal correspondant à l'absorption du réactif (bande à 592 nm) en fonction de la durée d'exposition. Ces deux valeurs sont directement proportionnelles à la concentration an acide acétique. Les résultats sont présentés sur les figures 8a et 8b.

## Revendications

1. Un procédé de piégeage d'aldéhydes, utilisant comme capteur un matériau sol-gel poreux essentiellement constitué
- d'unités d'un ou plusieurs premier(s) polyalcoxysilane(s) choisi(s) parmi les composés suivants : le (chlorométhyl)triéthoxysilane ; le 1,3-diméthyltetraméthoxydisiloxane ; l'éthyltriméthoxysilane ; le triéthoxy(éthyl)silane ; le triéthoxyméthylsilane ; le triéthoxy(vinyl)silane ; le triméthoxyméthylsilane ; le triméthoxy(vinyl)silane ; le tétraéthoxysilane ou le tétraméthoxysilane (TMOS) et
- d'unités d'un ou plusieurs second(s) polyalcoxysilane(s) choisi(s) parmi les composés suivants: le (N-(3-(triméthoxysilyl)propyl)éthylènediamine ; le 3-aminopropyltriéthoxysilane (APTES) et le 3-aminopropyltriméthoxysilane, dans un rapport molaire premier(s) polyalcoxysilane(s) / second(s) polyalcoxysilane(s) de 1/0,01 à 1/1 ledit matériau sol-gel incorporant une molécule sonde, dans lequel on met en contact un flux susceptible de contenir un aldéhyde avec ledit matériau sol-gel incorporant la molécule sonde, ou fait circuler un tel flux sur celui-ci.

2. Un procédé selon la revendication 1, **caractérisé en ce que** en outre, on procède à la détection des aldéhydes piégés sur les matériaux sol gel incorporant une molécule sonde.

3. Un procédé selon la revendication 1 ou 2, **caractérisé en ce que** en outre, on procède au dosage des aldéhydes piégés sur les matériaux sol gel incorporant une molécule sonde.

4. Un procédé selon l'une quelconque des revendications 1 à 3, **caractérisé en ce que** le rapport molaire premier(s) polyalcoxysilane(s) / second(s) polyalcoxysilane(s) est de 1/0,01 à 1/0,30.

5. Un procédé selon l'une quelconque des revendications 1 à 4, **caractérisé en ce que** le(s) premier (s) polyalcoxysilane(s) est (sont) choisi(s) parmi les composés suivants: le méthyltriméthoxysilane, le tétraéthoxysilane, le méthyltriéthoxysilane et le tétraméthoxysilane (TMOS).

6. Un procédé selon l'une quelconque des revendications 1 à 5, **caractérisé en ce qu'**il comprend
- un seul premier polyalcoxysilane ou
- un seul premier polyalcoxysilane et un seul second polyalcoxysilane.

7. Un procédé selon l'une quelconque des revendications 1 à 6, **caractérisé en ce que** le second polyalcoxysilane est le 3-aminopropyltriéthoxysilane.

8. Un procédé selon la revendication 7, **caractérisé en ce que** la molécule sonde est choisie parmi l'hydralazine, la 4-amino-3-penten-2-one, et la 2,4-dinitrophénylhydrazine.

9. Un procédé selon l'une quelconque des revendications 1 à 8, **caractérisé en ce que** le pourcentage pondéral de molécule-sonde est de 10 à 40% rapporté au poids total du matériau.

10. Un procédé selon l'une quelconque des revendications 3 à 9 **caractérisé en ce que** le dosage des aldéhydes piégés sur les matériaux sol-gel incorporant une molécule sonde est effectué par mesure des variations d'absorbance ou de fluorescence ou de luminescence de la molécule-sonde au cours du temps ou par mesure des variations d'absorbance ou de fluorescence ou de luminescence du produit de la réaction entre la molécule-sonde et les aldéhydes piégés, au cours du temps.

11. Un procédé selon l'une quelconque des revendications 1 à 10, **caractérisé en ce que** la molécule sonde est la 4-amino-3-penten-2-one.

12. Utilisation d'un matériau sol gel tel que défini à l'une quelconque des revendications 1 à 9 dans le piégeage d'aldéhydes ou dans leur détection.

## Patentansprüche

1. Verfahren zum Einfangen von Aldehyden, wobei als Fänger ein poröses Sol-Gel-Material verwendet wird, das im Wesentlichen aus:
- Einheiten von einem oder mehreren ersten Polyalkoxysilan(en), ausgewählt aus den folgenden Verbindungen: (Chlormethyl)triethoxysilan; 1,3- Dimethyltetramethoxydisiloxan; Ethyltrimethoxysilan; Triethoxy(ethyl)silan; Triethoxymethylsilan; Triethoxy(vinyl)silan; Trimethoxymethylsilan; Trimethoxy(vinyl)silan; Tetraethoxysilan oder Tetramethoxysilan (TMOS) und
- Einheiten von einem oder mehreren zweiten Polyalkoxysilan(en), ausgewählt aus den folgenden Verbindungen: (N-(3-(Trimethoxysilyl)propyl)ethylendiamin; 3-Aminopropyltriethoxysilan (APTES) und 3-Aminopropyltrimethoxysilan in einem Molverhältnis von erstem (ersten) Polyalkoxysilan(en)/zweitem (zweiten) Polyalkoxysilan(en) von 1/0,01 bis 1/1 besteht, wobei das Sol-Gel-Material ein Sondenmolekül beinhaltet, wobei ein Strom, der ein Aldehyd enthalten kann, mit dem Sol-Gel-Material, das das Sondenmolekül beinhaltet, in Kontakt gebracht wird oder ein solcher Strom auf diesem zirkulieren gelassen wird.

2. Verfahren nach Anspruch 1, **dadurch gekennzeichnet, dass** der Nachweis der eingefangenen Aldehyde ferner auf den Sol-Gel-Materialien, die ein Sondenmolekül beinhalten, erfolgt.

3. Verfahren nach Anspruch 1 oder 2, **dadurch gekennzeichnet, dass** die Quantifizierung der eingefangenen Aldehyde ferner auf den Sol-Gel-Materialien, die ein Sondenmolekül beinhalten, erfolgt.

4. Verfahren nach einem der Ansprüche 1 bis 3, **dadurch gekennzeichnet, dass** das Molverhältnis von erstem (ersten) Polyalkoxysilan(en)/zweitem (zweiten) Polyalkoxysilan(en) zwischen 1/0,01 und 1/0,30 liegt.

5. Verfahren nach einem der Ansprüche 1 bis 4, **dadurch gekennzeichnet, dass** das (die) erste (ersten) Polyalkoxysilan(e) aus den folgenden Verbindungen ausgewählt ist (sind): Methyltrimethoxysilan, Tetraethoxysilan, Methyltriethoxysilan und Tetramethoxysilan (TMOS).

6. Verfahren nach einem der Ansprüche 1 bis 5, **dadurch gekennzeichnet, dass** es
- ein einziges erstes Polyalkoxysilan oder
- ein einziges erstes Polyalkoxysilan und ein einziges zweites Polyalkoxysilan umfasst.

7. Verfahren nach einem der Ansprüche 1 bis 6, **dadurch gekennzeichnet, dass** das zweite Polyalkoxysilan 3-Aminopropyltriethoxysilan ist.

8. Verfahren nach Anspruch 7, **dadurch gekennzeichnet, dass** das Sondenmolekül aus Hydralazin, 4-Amino-3-penten-2-on und 2,4-Dinitrophenylhydrazin ausgewählt ist.

9. Verfahren nach einem der Ansprüche 1 bis 8, **dadurch gekennzeichnet, dass** der Gewichtsprozentanteil des Sondenmoleküls bezogen auf das Gesamtgewicht des Materials 10 bis 40 % beträgt.

10. Verfahren nach einem der Ansprüche 3 bis 9, **dadurch gekennzeichnet, dass** die Quantifizierung der auf den ein Sondermolekül beinhaltenden Sol-Gel-Materialien eingefangenen Aldehyde durch Quantifizieren Messen der Variationen von Absorbanz oder Fluoreszenz oder Lumineszenz des Sondenmoleküls im Zeitverlauf oder durch Messen der Variationen von Absorbanz oder Fluoreszenz oder Lumineszenz des Reaktionsprodukts aus dem Sondenmolekül und den eingefangenen Aldehyden im Zeitverlauf durchgeführt wird.

11. Verfahren nach einem der Ansprüche 1 bis 10, **dadurch gekennzeichnet, dass** das Sondenmolekül 4-Amino-3-penten-2-on ist.

12. Verwendung eines Sol-Gel-Materials wie in einem der Ansprüche 1 bis 9 definiert zum Einfangen von Aldehyden oder zu deren Nachweis.

## Claims

1. A process for trapping aldehydes using, as sensor, a porous sol-gel material consisting essentially of:
- units of one or more first polyalkoxysilane(s) selected from the following compounds: (chloromethyl)triethoxysilane; 1,3-dimethyltetramethoxydisiloxane; ethyltrimethoxysilane; triethoxy(ethyl)silane; triethoxymethylsilane; triethoxy(vinyl)silane; trimethoxymethylsilane; trimethoxy(vinyl)silane; tetraethoxysilane and tetramethoxysilane (TMOS); and
- units of one or more second polyalkoxysilane(s) selected from the following compounds: (N-(3-(trimethoxysilyl)propyl)ethylenediamine; 3-aminopropyltriethoxysilane (APTES) and 3-aminopropyltrimethoxysilane, in a first polyalkoxysilane(s)/second polyalkoxysilane(s) mole ratio of 1/0.01 to 1/1 said sol-gel material incorporating a probe molecule, wherein a stream that may contain aldehyde is placed in contact with the sol-gel material incorporating a probe molecule, or such a stream is made to circulate thereon.

2. The process as claimed in claim 1, **characterized in that** the detection of the aldehydes trapped on the sol-gel materials incorporating a probe molecule is also performed.

3. The process as claimed in either of claims 1 or 2, **characterized in that** the assay of the aldehydes trapped on the sol-gel materials incorporating a probe molecule is also performed.

4. The process as claimed in any one of claims 1 to 3, **characterized in that** the first polyalkoxysilane(s)/second polyalkoxysilane(s) mole ratio is from 1/0.01 to 1/0.30.

5. The process as claimed in any one of claims 1 to 4, **characterized in that** the first polyalkoxysilane(s) are chosen from the following compounds: methyltrimethoxysilane, tetraethoxysilane, methyltriethoxysilane and tetramethoxysilane (TMOS).

6. The process as claimed in any one of claims 1 to 5, **characterized in that** it comprises:
- a single first polyalkoxysilane, or
- a single first polyalkoxysilane and single second polyalkoxysilane.

7. The process as claimed in any one of claims 1 to 6, **characterized in that** the second polyalkoxysilane is 3-aminopropyltriethoxysilane.

8. The process as claimed in any one of claims 1 to 7, **characterized in that** the probe molecule is chosen from hydralazine, 4-amino-3-penten-2-one, and 2,4-dinitrophenylhydrazine.

9. The process as claimed in any one of claims 1 to 8, **characterized in that** the weight percentage of probe molecule is from 10% to 40% relative to the total weight of the material.

10. The process as claimed in any one of claims 3 to 9, **characterized in that** the assay of the aldehydes trapped on the sol-gel material incorporating a probe molecule is performed using a measurement of either the variations in absorbance, fluorescence or luminescence of the probe molecule over time or the variations in absorbance, fluorescence or luminescence of the product of the reaction between the probe molecule and the aldehydes trapped over time.

11. The process as claimed in any one of claims 1 to 10, **characterized in that** the probe molecule is the 4-amino-3-penten-2-one.

12. The use of a sol-gel material as define in any one of claims 1 to 9 in the trapping of aldehyde or in their detection.
